# EUROPEAN PATENT APPLICATION

(11) **EP 4 674 821 A1**
(43) Date of publication of application: **07.01.2026**
(21) Application number: 25169663.9
(22) Date of filing: 10.04.2025
(51) Int. Cl.: C02F 9/00, B01D 53/26, C02F 1/20, C02F 1/40, C02F 1/66, C02F 3/28, C02F 11/04, C02F 101/16, C02F 103/18, C02F 103/32, C12M 1/107, C12M 1/00

(54) **SYSTEM FOR MAXIMIZING PRODUCTION OF BIO-GAS AND MINIMIZING EMISSION OF PROCESS WASTES**

(30) Priority: 04.07.2024 KR 20240088038
(71) Applicant: Na, Min Soo, Hwaseong-si, Gyeonggi-do (KR)
(72) Inventor: Na, Min Soo, Hwaseong-si, Gyeonggi-do (KR)
(74) Representative: Cabinet Chaillot

(57) **Abstract**

A system for producing bio-gas from food waste comprising:

- a crusher,

- a dehydrator that separates crushed food waste in a wastewater fraction and a sludge fraction,

- an oil remover configured to remove oil from the food wastewater;

- a primary dryer configured to dry-treat the sludge

- a primary anaerobic digester to treat food wastewater discharged from the oil remover,

- a secondary anaerobic digester to treat dried material discharged from the primary dryer

- a maturation tank to circulate the digested sludge between the primary digester and the secondary digester

- an ammonia stripping reactor configured that adds an alkaline agent to the digested sludge is discharged from the maturation tank,

- a secondary dryer to dry-treat the sludge discharged from the ammonia stripping reactor;

- deodorizing units to incinerate waste gases discharged from the primary and secondary dryer.

The system that recycles high-concentration organic food wastewater and organic sludge generated during the food waste treatment process within the process itself to increase bio-gas production and minimize process waste. The system maximizes bio-gas production and minimizes waste discharge by constructing key facilities, such as anaerobic digesters, dryers, and deodorizing boilers, in a two-stage configuration and integrating these facilities into an interconnected process. By implementing this system, dried materials generated in the dryers, as well as digested slurry and condensed wastewater generated during the treatment process, may be recycled and recovered within the process. As a result, energy costs may be reduced by increasing bio-gas production, and minimizing external discharge of process waste outsourcing treatment costs may be reduced.

## Description

### CROSS-REFERENCE TO RELATED APPLICATION

This application claims priority to Korean Patent Application No. 10-2024-0088038 filed on July 4, 2024 and all the benefits accruing therefrom under 35 U.S.C. §119, the contents of which are incorporated by reference in their entirety.

### BACKGROUND

The present disclosure relates to a system for maximizing the production of bio-gas and minimizing the emission of process wastes, and more particularly, to a system that recycles high-concentration organic food waste leachate and organic sludge generated during the food waste treatment process within the process to increase bio-gas production and minimize process waste.

Generally, food waste has been recycled by processing it into reusable resources, which involves reducing its volume through crushing, dehydrating, or drying, and then repurposing it as compost or animal feed.

That is, since food waste has a high moisture content and generates odors as it decomposes, it is typically processed through drying and carbonization to produce solid materials with an extremely low moisture content. These solids are then utilized in various applications, such as compost, animal feed, solid fuel, or landfill cover.

For example, as part of the process to reduce the moisture content of food waste, dehydrating is performed. The dehydrated food waste is then dried to further lower its moisture content. Subsequently, the dried food waste is crushed, and foreign substances, such as metal contaminants, are removed through a sifting process before being utilized as fertilizer or other recyclable materials.

However, such systems have significant drawbacks, as they generate leachate and condensate during the dehydrating and drying stages, requiring substantial costs for treatment and also posing environmental pollution risks. Additionally, the drying stage necessitates a continuous heat source to sustain high temperatures for food waste processing, resulting in considerable energy consumption.

That is, industrial process systems for food waste treatment, including pretreatment steps such as crushing and dehydrating, followed by drying, require substantial amounts of thermal and electrical energy. The high energy costs directly lead to an increase in the unit production cost, placing a significant financial burden on businesses. Furthermore, most of the wastewater and waste materials generated during the process must be outsourced for disposal, making the process economically disadvantageous.

Meanwhile, methods have been developed to recycle biogas produced during anaerobic digestion of food waste, either by using it as a fuel for gas generators or converting it into electrical energy. However, these approaches still leave residual waste that requires further processing. In addition, the dried material after processing contains a high nitrogen content, making it difficult to achieve high-quality feed production.

### [Prior Art Document]

(Patent Document 1) Korean Patent No. 10-1367745
(Patent Document 2) Korean Patent No. 10-1467695
(Patent Document 3) Korean Patent No. 10-1847082
(Patent Document 4) Korean Patent No. 10-2648024

### SUMMARY

The present disclosure provides a system for maximizing production of bio-gas and minimizing emission of process wastes is provided as an object of the present invention. In the novel system according to the present invention, key facilities, such as anaerobic digesters, dryers, and deodorizing boilers, are constructed in a two-stage configuration and integrated into an interconnected process. This system enables the recycling and recovery of dried materials from the dryer, as well as digested slurry and condensed wastewater generated during processing, within the process itself, thereby increasing bio-gas production and reducing energy costs. In addition, by minimizing the external discharge of process wastes, outsourcing disposal costs may be reduced.

To achieve the above objectives, the system for maximizing bio-gas production and minimizing process waste discharge provided by the present invention has the following features.

The system for maximizing production of bio-gas and minimizing emission of process wastes includes: a food waste pretreatment part including: a crusher configured to crush food waste, a dehydrator configured to dewater food waste, and an oil remover configured to remove oil from food waste; a primary dryer configured to dry-treat organic sludge discharged from the dehydrator of the food waste pretreatment part; a primary digester configured to anaerobically treat food wastewater, which is discharged from the oil remover of the food waste pretreatment part, and digested sludge, which is circulated from a maturation tank, to produce bio-gas; a secondary digester configured to anaerobically treat dried material, which is discharged from the primary dryer, and digested sludge, which is circulated from the maturation tank, to produce bio-gas; the maturation tank configured to mature digested sludge, which is discharged from the primary digester and the secondary digester, while circulating the digested sludge with the primary digester and the secondary digester; an ammonia stripping reactor configured to strip ammonia by adding an alkaline agent to the digested sludge, which is discharged from the maturation tank, thereby increasing a pH to at least about 11; a secondary dryer configured to dry-treat the digested sludge discharged from the ammonia stripping reactor; a primary deodorizing boiler configured to incinerate waste gas discharged from the primary dryer; and a secondary deodorizing boiler configured to incinerate waste gas discharged from the secondary dryer.

In an embodiment, the system may further include a gas holder configured to collect and pretreat bio-gas produced from the primary digester and bio-gas produced from the secondary digester, and to supply the pretreated bio-gas as fuel to the primary deodorizing boiler and the secondary deodorizing boiler.

In an embodiment, the primary dryer and the secondary dryer may utilize steam supplied from the primary deodorizing boiler and the secondary deodorizing boiler, respectively, as a heat source.

In an embodiment, the ammonia stripping reactor may be configured to receive a portion of digested treated effluent discharged from the maturation tank and to perform ammonia stripping treatment while the digested sludge and the digested treated effluent are mixed together.

In an embodiment, the secondary digester may be configured to receive a portion of the condensed wastewater generated from the waste gas discharged from the primary dryer and the secondary dryer and to perform anaerobic digestion treatment while the condensed wastewater is mixed with the dried material.

In an embodiment, the system may further include a crusher configured to crush the dried material discharged from the primary dryer; a screening device configured to screen the dried material discharged from the primary dryer; and a mixing tank configured to mix the dried material discharged from the screening device with condensed wastewater and to transfer the mixture to the secondary digester.

### BRIEF DESCRIPTION OF THE DRAWINGS

Exemplary embodiments can be understood in more detail from the following description taken in conjunction with the accompanying drawings, in which:
FIG. 1 is a schematic view of a system for maximizing production of bio-gas and minimizing emission of process wastes according to an embodiment of the present invention; and
FIG. 2 is a schematic view of a system for maximizing production of bio-gas and minimizing emission of process wastes according to another embodiment of the present invention.

### DETAILED DESCRIPTION OF EMBODIMENTS

Hereinafter, it should be understood that the exemplary embodiments herein are provided only for the illustrative purpose, and various modifications of the embodiments are reproduced. However, detailed descriptions related to well-known functions or configurations will be ruled out in order not to unnecessarily obscure subject matters of the present disclosure, and the detailed descriptions and specific illustrations will be omitted. In addition, the shapes and the sizes of elements in accompanying drawings will be exaggerated for more apparent description.

Hereinafter, the present invention will be described in detail with reference to the accompanying drawings.

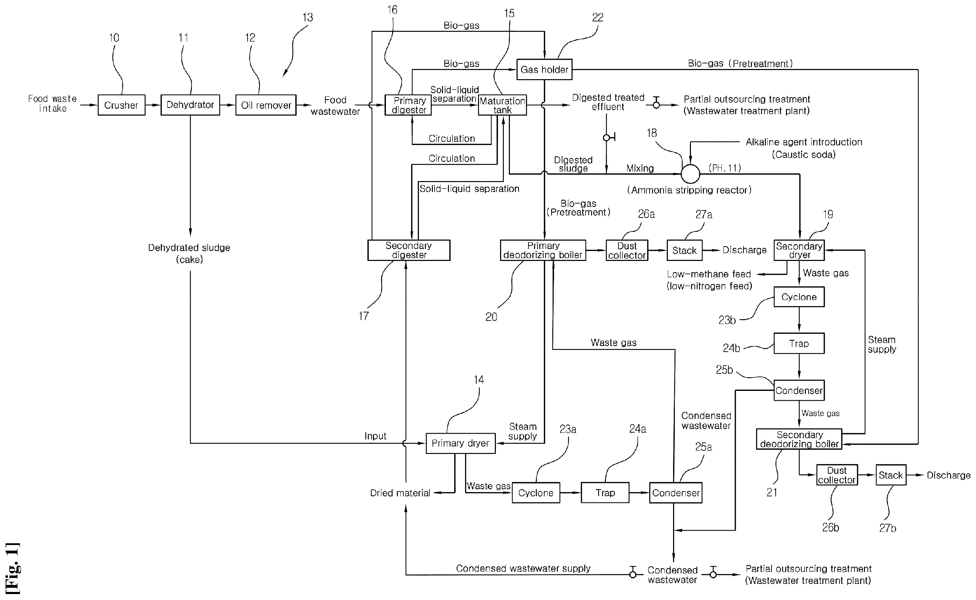

FIG. 1 is a schematic view of a system for maximizing production of bio-gas and minimizing emission of process wastes according to an embodiment of the present invention.

As shown in FIG. 1, the system for maximizing production of bio-gas and minimizing emission of process wastes may include a food waste pretreatment part 13 as a means for the pretreatment of food waste.

The food waste pretreatment part 13 may include a crusher 10, a dehydrator 11, and an oil remover 12, which are sequentially arranged to treat food waste.

The crusher 10 may crush food waste that is clumped together in lump form. The dehydrator 11 may extract moisture from the food waste, and the oil remover 12, functioning as a type of three-phase separator, may eliminate oil contained in the food waste.

Accordingly, food waste introduced from the outside may sequentially pass through the crusher 10, the dehydrator 11, and the oil remover 12. Through this process, the food waste may be finely crushed while its moisture and oil content are effectively removed.

The organic sludge discharged from the dehydrator 11 may be transferred to a primary dryer 14 for dry-treating, and the food wastewater discharged from the dehydrator 11 may pass through the oil remover 12 before being transferred to a primary digester 16 for anaerobic digestion treatment.

Additionally, the system for maximizing production of bio-gas and minimizing emission of process wastes may include the primary dryer 14 as a means for dry-treating the organic sludge, which is discharged from the dehydrator 11 of the food waste pretreatment part 13.

The primary dryer 14 may function to dry-treat the organic sludge discharged from the dehydrator 11, and the dried material from the primary dryer 14 may then be transferred to a secondary digester 17 for bio-gas production.

Furthermore, the waste gas discharged from the primary dryer 14, which contains high-concentration N₂ gas, may pass through a cyclone 23a and a trap 24a, where impurities and oil are filtered out. The waste gas that has passed through the cyclone 23a and the trap 24a may be transferred to a condenser 25a for cooling treatment and then be transferred to a primary deodorizing boiler 20 for deodorization treatment. Afterward, the deodorized gas may pass through a dust collector 26a and a stack 27a and released into the atmosphere.

Here, a portion of the condensed wastewater discharged from the condenser 25a may be outsourced for treatment, and the remaining condensed wastewater may be mixed with the dried material discharged from the primary dryer 14 and transferred together to the secondary digester 17 for treatment.

Additionally, the system for maximizing production of bio-gas and minimizing emission of process wastes may include the primary digester 16 as a means for producing bio-gas by anaerobically treating the food wastewater discharged from the oil remover 12 in the food waste pretreatment part 13 and the digested sludge circulated from a maturation tank 15.

The primary digester 16 may be a mesophilic digester in which the internal temperature is maintained at approximately 37 to 42°C. The primary digester 16 may anaerobically treat not only the digested sludge circulated between the maturation tank 15 but also the high-concentration organic food wastewater, from which oil has been removed, supplied from the oil remover 12.

For example, when digested sludge and high-concentration organic food wastewater are introduced into the primary digester 16, the digested sludge and high-concentration organic food wastewater may remain inside for about 25 to about 35 days while maintaining a pH of about 7.0 to about 7.6. The bio-gas generated through this retention and fermentation process, may be collected in an upper part of the digester, digested treated effluent may be collected in an upper liquid layer of the digester, and the heavier digested sludge may be collected in a lower layer of the digester.

The bio-gas discharged from the primary digester 16 may be transferred to a gas holder 22, while the digested treated effluent and digested sludge may be ultimately transferred to the maturation tank 15.

Additionally, the system for maximizing production of bio-gas and minimizing emission of process wastes may include the secondary digester 17 as a means for producing bio-gas by anaerobically treating the dried material discharged from the primary dryer 14 and the digested sludge circulated from the maturation tank 15.

The secondary digester 17 may be a mesophilic digester in which an internal temperature is maintained at approximately 37 to 42°C. The secondary digester 17 may anaerobically treat not only the dried material supplied from the primary dryer 14 but also the digested sludge circulated between the maturation tank 15.

For example, when digested sludge and high-concentration organic food wastewater are introduced into the primary digester 17, the digested sludge and high-concentration organic food wastewater may remain inside for a predetermined time, for example, about 25 to about 35 days while maintaining a pH of about 7.0 to about 7.6. The bio-gas generated through this retention and fermentation process, may be collected in the upper portion of the digester, the digested treated effluent may be collected in the upper liquid layer of the digester, and the heavier digested sludge may be collected in the lower layer of the digester.

The bio-gas discharged from the secondary digester 17 may be transferred to the gas holder 22, while the digested treated effluent and digested sludge may be ultimately transferred to the maturation tank 15.

In particular, when the secondary digester 17 receives dried material from the primary dryer 14, it may also receive condensed wastewater generated from the waste gas discharged from the primary dryer 14, i.e., the condensed wastewater generated through the gas condensation process in the condenser 25a, as well as the condensed wastewater generated from the waste gas discharged from the secondary dryer 19, i.e., the condensed wastewater generated through the gas condensation process in the condenser 25b. Accordingly, the secondary digester 17 may anaerobically treat the dried material and the condensed wastewater that are mixed together.

At this point, the condensed wastewater, which is distributed through two separate lines from the two condensers 25a and 25b, may merge into a single line, and the condensed wastewater flowing through the single line may then be redistributed. Then, a portion of the condensed wastewater may be supplied to the secondary digester 17, while the remaining portion may be outsourced for treatment at a wastewater treatment plant or a wastewater treatment process.

As a preferred embodiment, the dried material supplied to the secondary digester 17, specifically, the dried material provided from the primary dryer 14, may undergo a crushing and sifting process, as well as a mixing process and then be transferred to the secondary digester 17 to improve digestion efficiency.

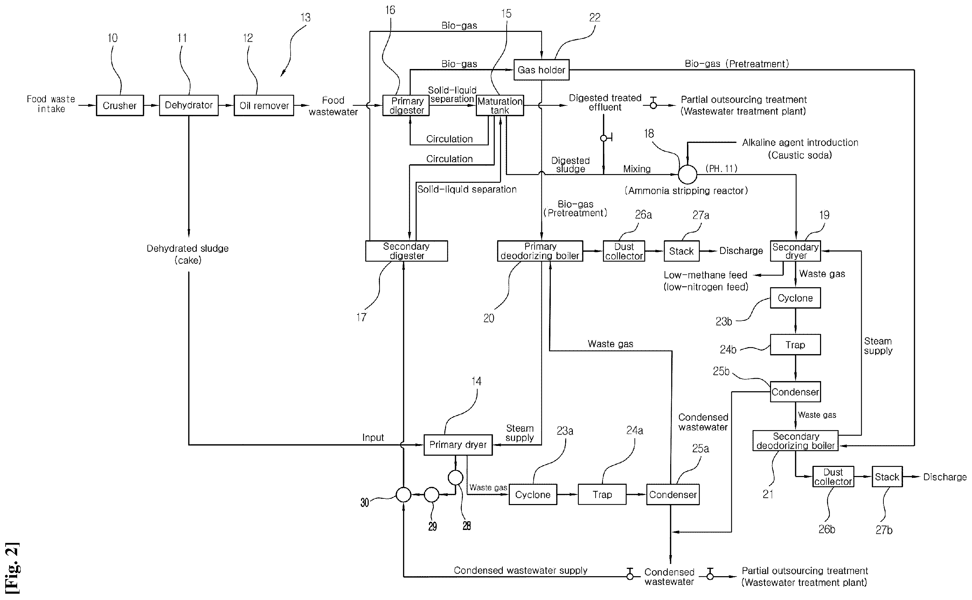

As shown in FIG. 2, for this purpose, a crusher 28, a sifting machinery 29, and a mixing tank 30 may be sequentially installed on a dried material discharge-side line of the primary dryer 14, specifically, the dried material discharge-side line leading to the secondary digester 17. At this point, the mixing tank 30 may be connected to a line through which condensed wastewater generated through the gas condensation process in the condenser 25a, as well as condensed wastewater generated through the gas condensation process in the condenser 25b are supplied and connected to the secondary digester 17.

Accordingly, the dried material discharged from the primary dryer 14 may be crushed to an appropriate size while passing through the crusher 28. Consequently, the crushed material may pass through the sifting machinery 29, for example, a rotary screen, where it may be screened to an appropriate size then be transferred to the mixing tank 30.

Subsequently, in the mixing tank 30, the dried material introduced from the sifting machinery 29 and the condensed wastewater supplied from the condensers 25a and 25b may be mixed. The mixed dried material and condensed wastewater may then be transferred to the secondary digester 17 for anaerobic digestion treatment. Since the dried material that has undergone a crushing process and a sifting process is anaerobically digested in the secondary digester 17, digestion treatment efficiency may be further improved.

Additionally, the system for maximizing production of bio-gas and minimizing emission of process wastes may include the maturation tank 15 as a means for circulating digested sludge with the primary digester 16 and the secondary digester 17, while maturing the digested sludge, which is discharged from the primary digester 16 and the secondary digester 17.

The maturation tank 15 may perform fermentation maturation and solid-liquid separation processes while retaining the digested sludge and filtrate discharged from the primary digester 16 and the secondary digester 17 for a certain period.

Moreover, the digested sludge may be recirculated among the maturation tank 15, the primary digester 16, and the secondary digester 17 for a predetermined period while undergoing fermentation and maturation processes. As a result, these repeated fermentation and maturation processes may maximize bio-gas production.

The digested sludge discharged from the maturation tank 15 after undergoing the maturation process may be transferred to the ammonia stripping reactor 18, while a portion of the digested treated effluent may be transferred to the ammonia stripping reactor 18 for use, and the remaining portion may be outsourced for treatment at a wastewater treatment plant or similar facility.

Additionally, the system for maximizing production of bio-gas and minimizing emission of process wastes may include the ammonia stripping reactor 18 as a means for stripping ammonia by adding an alkaline agent to the digested sludge, which is discharged from the maturation tank 15, thereby increasing in pH to about 11 or more.

The ammonia stripping reactor 18 may function to increase the pH of the digested sludge and the digested treated effluent introduced together to about 11 or more, through the addition of an alkaline agent and agitation.

For this purpose, the ammonia stripping reactor 18 may receive the digested sludge and the digested treated effluent along with about 1 wt% to about 5 wt% of an alkaline agent, such as liquid caustic soda, an alkali metal compound, or an alkaline earth metal compound. The mixture of the introduced digested sludge, digested treated effluent, and a mixture of alkaline agents, that is, a mixture with a pH of about 11 or more, may undergo decomposition, diffusion, and breakdown reactions in the ammonia stripping reactor 18 and may be transferred to the secondary dryer 19.

For example, in the ammonia stripping reactor 18, a denitrification treatment process may be performed on the introduced digested sludge, digested treated effluent, and the alkaline chemicals.

That is, digested sludge, digested treated effluent, and the alkaline chemicals may be introduced into the ammonia stripping reactor 18 and mixed. At this time, the internal conditions of the ammonia stripping reactor 18 may be set to maintain relatively high temperatures and a strong alkaline state with a pH of about 11 or more.

Accordingly, as the digested sludge and the digested treated effluent may be introduced into the ammonia stripping reactor 18, alkaline chemicals may also be introduced, and continuous rotational agitation may be performed in the ammonia stripping reactor 18 in a state in which the internal conditions are maintained at a predetermined temperature and a strong-alkaline state with a pH of about 11 or more, while inducing physical changes and performing the mixing process. As a result, the nitrogen compounds contained in the digested sludge and the digested treated effluent may undergo the degassing process, in which ammonium (NH₄⁺) ions rapidly convert into ammonia (NH₃), thereby reducing the total nitrogen content within a short period.

Additionally, a portion of the digested treated effluent, which is transferred from the maturation tank 15 to the ammonia stripping reactor 18, may be outsourced for treatment.

At this point, the ammonia stripping process is a type of physicochemical nitrogen removal method for substances containing large amounts of food waste, food wastewater, ammonia, and ammonium ions. This process may involve adding alkaline chemicals, such as caustic soda, to raise the pH to about 11 or more, and maintaining a predetermined temperature, which allows ammonia to be reduced and degassed as nitrogen gas.

Furthermore, the system for maximizing production of bio-gas and minimizing emission of process wastes may include the secondary dryer 19 as a means for dry-treating the digested sludge discharged from the ammonia stripping reactor 18.

In the secondary dryer 19, the digested sludge may be dried, and the ammonia stripping reaction may continuously proceed, thereby enabling denitrification treatment.

For example, the digested sludge, the digested treated effluent, and alkaline chemicals may be introduced into the secondary dryer 19 as a mixture. The drying conditions inside the secondary dryer 19 may be maintained at a high temperature in a strong-alkaline state with a pH of about 11 or more.

Accordingly, continuous rotational agitation drying may be performed in the secondary dryer 19 in a state in which the internal conditions are maintained at a high temperature and the pH of about 11 or more, while inducing physical changes and performing the dry-treating, which enables the removal of total nitrogen compounds contained in the dried digested sludge.

At this point, ammonia (NH₃) degassed from the dried material through the ammonia stripping process inside the secondary dryer 19 may be transferred along with the dryer waste gas to the secondary deodorizing boiler 21 for treatment. Additionally, the dried material discharged from the secondary dryer 19, which contains a low concentration of total nitrogen compounds, may be used as feed, compost, or solid fuel.

Accordingly, the denitrified dried material discharged from the secondary dryer 19, such as organic dried feed containing a low concentration of total nitrogen compounds, that is, low-methane feed (low-nitrogen feed), may undergo a crushing and sifting process before being sold as compost, solid fuel, or other products.

Additionally, the waste gas discharged from the secondary dryer 19, which contains a high concentration of N₂gas, may pass through the cyclone 23b and the trap 24b, where impurities and oil in the waste gas may be filtered out. The waste gas that has passed through the cyclone 23b and the trap 24b may then be transferred to the condenser 25b for cooling treatment and transferred to the secondary deodorizing boiler 21 for deodorization treatment. Afterward, the deodorized gas may pass through the dust collector 26b and the stack 27b and released into the atmosphere.

Furthermore, the system for maximizing production of bio-gas and minimizing emission of process wastes may include the primary deodorizing boiler 20 and the secondary deodorizing boiler 21 as means for incinerating waste gas containing odorous gases.

The primary deodorizing boiler 20 may be installed on the waste gas discharge line of the primary dryer 14 and may serve to incinerate the waste gas discharged from the primary dryer 14.

The secondary deodorizing boiler 21 may be installed on the waste gas discharge line of the secondary dryer 19 and may serve to incinerate the waste gas discharged from the secondary dryer 19.

The waste gas incinerated in the primary deodorizing boiler 20 and the secondary deodorizing boiler 21 may be purified through the dust collectors 26a and 26b and the stacks 27a and 27b and then be released into the atmosphere.

As a preferred embodiment, the present invention provides a system that utilizes the bio-gas produced in the primary digester 16 and the secondary digester 17 as fuel for operating the facilities.

For this purpose, gas transport lines may be connected between the primary digester 16, the secondary digester 17, and the primary deodorizing boiler 20 and the secondary deodorizing boiler 21, respectively, and a gas holder 22 may be installed on the connected lines.

Accordingly, the bio-gas discharged from the primary digester 16 and the secondary digester 17 may be collected and pretreated in the gas holder 22 and supplied as fuel to the primary deodorizing boiler 20 and the secondary deodorizing boiler 21.

Since the bio-gas produced in the digesters is utilized as fuel for operating the facilities, energy costs may be significantly reduced.

Additionally, steam transport lines may be connected between the primary deodorizing boiler 20 and the secondary deodorizing boiler 21 and the primary dryer 14 and the secondary dryer 19, respectively. Through these lines, steam generated in the primary deodorizing boiler 20 and the secondary deodorizing boiler 21 may be supplied to the primary dryer 14 and the secondary dryer 19, where it is used as a heat source for drying.

Accordingly, the overall food waste treatment process in the system for maximizing production of bio-gas and minimizing emission of process wastes will be described as follows.

As the food waste sequentially passes through the crusher 10, the dehydrator 11, and the oil remover 12 of the food waste pretreatment part 13, it may be finely crushed, and the moisture and oil contained in the food waste may be removed.

The organic sludge discharged from the dehydrator 11 may be introduced into the primary dryer 14 for dry-treating, after which the dried material may be introduced into the secondary digester 17 in a state mixed with condensed wastewater. Additionally, the high-concentration food wastewater discharged from the oil remover 12 may be introduced into the primary digester 16.

At this point, the waste gas discharged from the primary dryer 14 may be deodorized in the primary deodorizing boiler 20 and released into the atmosphere.

In the primary digester 16 and the secondary digester 17, bio-gas may be generated through anaerobic digestion treatment. The bio-gas may then be collected in the gas holder 22 and supplied as fuel to the primary deodorizing boiler 20 and the secondary deodorizing boiler 21. Meanwhile, the digested sludge and the digested treated effluent discharged from the primary digester 16 and the secondary digester 17 may be matured in the maturation tank 15 and introduced into the ammonia stripping reactor 18.

In addition, the waste gas discharged from the second dryer 19 may be deodorized in the secondary deodorizing boiler 21 and released into the atmosphere.

In the ammonia stripping reactor 18, a mixture of the digested sludge and the digested treated effluent with minimized nitrogen content may be produced through the ammonia stripping treatment. The produced mixture of the digested sludge and the digested treated effluent may then be introduced into the secondary dryer 19 for dry-treating. Meanwhile, the high-quality, low-nitrogen dried material discharged from the secondary dryer 19 may be sold as feed or compost. Additionally, the waste gas discharged from the secondary dryer 19 may be deodorized in the secondary deodorizing boiler 21 and released into the atmosphere.

In this manner, the bio-gas (methane) generated when introducing high-concentration organic food wastewater in the primary digester 16 may be supplied as fuel to the primary deodorizing boiler 20 after pretreatment. At this point, the steam generated in the primary deodorizing boiler 20 may be supplied as a heat source to the primary dryer 14 for dry-treating of the organic sludge.

Additionally, the organic dried material discharged from the primary dryer 14 may be mixed at a predetermined ratio with the condensed wastewater generated during the waste gas condensation process in both the primary and secondary dryers and be introduced into the secondary digester 17 for anaerobic digestion treatment.

Additionally, the bio-gas generated after digestion in the secondary digester 17 may be collected in the gas holder along with the bio-gas generated after digestion in the primary digester 16, and after pretreatment, the collected bio-gas may be used as fuel for the primary deodorizing boiler 20 and the secondary deodorizing boiler 21. The remaining bio-gas may undergo a refinement process to be converted into city gas for sale or supplied as fuel for a generator, with the electricity generated also being available for sale.

Furthermore, the digested treated effluent discharged from the primary digester 16 and the secondary digester 17 may undergo solid-liquid separation in the maturation tank 15. The anaerobic digested sludge may be transferred back to the primary digester 16 and the secondary digester 17, and excess digested sludge may be introduced into the ammonia stripping reactor 18 along with a predetermined ratio of digested treated effluent. In the ammonia stripping reactor 18, the mixture may be sufficiently agitated under alkaline conditions with the pH of about 11 or more to facilitate the degassing of ammonia (NH₃) gas and then be transferred to the secondary dryer 19 for dry-treating.

Additionally, the high-quality organic dried material discharged from the secondary dryer 19, which contains low nitrogen content, may be used as a compost raw material. The condensed wastewater discharged during the waste gas condensation process in the secondary dryer 19, which also contains low nitrogen content, may be resupplied as blending water for the primary dryer 14.

Moreover, the waste gas generated in the secondary dryer 19 may be incinerated in the secondary deodorizing boiler 21 for odor removal. The steam produced from the bio-gas combustion in the secondary deodorizing boiler 21 may be supplied as a heat source for the secondary dryer 19.

Accordingly, by utilizing a system that applies to this series of treatment processes, high-concentration organic food wastewater may be treated through a dual-stage process, maximizing the production of bio-gas as a renewable energy source. Additionally, the bio-gas may be utilized as fuel for the deodorizing boilers, and remaining bio-gas may be refined and sold to generate profit. Furthermore, the discharge amount of process waste generated during treatment may be minimized, which contributes to the reduction of greenhouse gas emissions and the improvement of environmental sustainability, and also significantly reduces high waste treatment costs.

The system for maximizing production of bio-gas and minimizing emission of process wastes provided by the present invention may have the following effects:
1) By implementing a system that recycles high-concentration organic food wastewater and organic sludge within the process, biogas production may be increased. The biogas produced through this process may be utilized as an internal energy source for operating process facilities, thereby reducing energy costs. Additionally, minimizing the waste and condensed wastewater discharged from the treatment process may reduce outsourcing disposal costs.
2) High-quality compost with a low nitrogen content may be produced and sold, contributing to corporate profitability.
3) The utilization of biogas as a renewable energy source may reduce greenhouse gas emissions and air pollutants, thereby improving air quality.

As described above, while the embodiments of the present disclosure have been described with reference to the specific embodiments, it will be apparent to those skilled in the art that various changes and modifications may be made without departing from the spirit and scope of the invention as defined in the following claims.

## Claims

1. A system for maximizing production of a bio-gas and minimizing emission of process wastes, the system comprising:
a food waste pretreatment part (13) comprising:
a crusher (10) configured to crushing food waste,
a dehydrator (11) configured to dehydrate the food waste, and
an oil remover (12) configured to remove oil from the food waste;
a primary dryer (14) configured to dry-treat organic sludge discharged from the dehydrator (11) of the food waste pretreatment part (13);
a primary digester (16) configured to anaerobically treat food wastewater, which is discharged from the oil remover (12) of the food waste pretreatment part (13), and digested sludge, which is circulated from a maturation tank (15), to produce the bio-gas;
a secondary digester (17) configured to anaerobically treat dried material, which is discharged from the primary dryer (14), and digested sludge which is circulated from the maturation tank (15), to produce the bio-gas;
the maturation tank (15) configured to circulate the digested sludge with the primary digester (16) and the secondary digester (17) while maturing the digested sludge, which is discharged from the primary digester (16) and the secondary digester (17);
an ammonia stripping reactor (18) configured to strip ammonia by adding an alkaline agent to the digested sludge, which is discharged from the maturation tank (15), thereby increasing in pH to about 11 or more;
a secondary dryer (19) configured to dry-treat the digested sludge discharged from the ammonia stripping reactor (18);
a primary deodorizing boiler (20) configured to incinerate a waste gas discharged from the primary dryer (14); and
a secondary deodorizing boiler (21) configured to incinerate a waste gas discharged from the secondary dryer (19).

2. The system according to claim 1, further comprising:
a gas holder (22) configured to collect and pretreat the bio-gas produced from the primary digester (16) and the bio-gas produced from the secondary digester (17), and to supply the pretreated bio-gas as fuel to the primary deodorizing boiler (20) and the secondary deodorizing boiler (21).

3. The system according to claim 1, wherein the primary dryer (14) and the secondary dryer (19) utilize steam supplied from the primary deodorizing boiler (20) and the secondary deodorizing boiler (21), respectively, as a heat source.

4. The system according to claim 1, wherein the ammonia stripping reactor (18) is configured to receive a portion of digested treated effluent discharged from the maturation tank (15) and to perform ammonia stripping treatment in a state in which the digested sludge and the digested treated effluent are mixed together.

5. The system according to claim 1, wherein the secondary digester (17) is configured to receive a portion of the condensed wastewater generated from the waste gas discharged from the primary dryer (14) and the secondary dryer (19) and to perform anaerobic digestion treatment in a state in which the condensed wastewater is mixed with the dried material.

6. The system according to claim 1, further comprising:
a crusher (28) configured to crush the dried material discharged from the primary dryer (14);
a sifting machinery (29) configured to sift the dried material discharged from the primary dryer (14); and
a mixing tank (30) configured to mix the dried material discharged from the screening device (29) with condensed wastewater and to transfer the mixture to the secondary digester (17).
